# EUROPÄISCHE PATENTSCHRIFT

(11) **EP 2 457 601 B1**
(45) Veröffentlichungstag und Bekanntmachung des Hinweises auf die Patenterteilung: **22.07.2015**
(21) Anmeldenummer: 11185357.8
(22) Anmeldetag: 17.10.2011
(51) Int. Cl.: A61L 31/02, A61L 31/12, A61L 31/14, A61L 31/18

(54) **Markerkomposit und medizinisches Implantat mit einem Röntgenmarker**
Marker composite and medical implant comprising an x-ray marker
Composite de marqueur et implant médical doté d'un marqueur de radiographie

(30) Priorität: 08.11.2010 US 410976 P
(43) Veröffentlichungstag der Anmeldung: 30.05.2012
(73) Patentinhaber: Biotronik AG, 8180 Bülach (CH)
(72) Erfinder: Götzen, Nils, 19057 Schwerin (DE); Mirabelli, Anna, 18055 Rostock (DE); Schoof, Andre, 18209 Bad Doberan (DE); Bayer, Ullrich, 18211 Admannshagen-Bargeshagen (DE); Sadler, Dirk, 18109 Rostock (DE); Riedmüller, Johannes, 90411 Nürnberg (DE)
(74) Vertreter: Lindner-Vogt, Karin L.

(56) Entgegenhaltungen:
- EP-A2- 0 824 900
- DE-A1-102006 038 238
- DE-A1-102008 043 642

## Beschreibung

Die Erfindung betrifft ein Markerkomposit für medizinische Implantate aus einem biokorrodierbaren metallischen Werkstoff sowie ein medizinisches Implantat aus einem biokorrodierbaren metallischen Werkstoff mit einem Röntgenmarker, der aus dem Markerkomposit besteht oder hergestellt wurde.

### Stand der Technik und Hintergrund der Erfindung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit biokorrodierbaren Basislegierungen, insbesondere des Magnesiums.

Zur radiologischen intra- und postoperativen Positionsüberwachung werden Implantate, sofern sie nicht bereits aus einem ausreichend radioopaken Material bestehen, mit mindestens einem Marker versehen. Die Röntgensichtbarkeit des Markers hängt von seinen Abmessungen und Röntgenabsorptionskoeffizienten ab. Letzterer ist wiederum abhängig vom Energiebereich der Röntgenstrahlung: im medizinischen Bereich liegt dieser in der Regel bei 60 bis 120 keV, bei koronarer Anwendung wird typischerweise im Bereich von 60 bis 100 keV gearbeitet. Mit steigender Ordnungszahl im Periodensystem und steigender Dichte des Materials wird der Röntgenabsorptionskoeffizienten in der Regel größer. Die Gegenwart des Markers sollte nicht die Funktionalität des Implantats einschränken und/oder Ausgangspunkt für Entzündungs- oder Abstoßungsreaktionen des Körpers sein. Als Markerwerkstoffe werden herkömmlich beispielsweise Edelmetalle, wie Gold und Platin, eingesetzt.

Die Marker liegen (i) als Vollmaterial, z. B. in Form einer Beschichtung, eines Bandes, eines Inlays oder eines mit dem Implantat fest verbundenen Formkörpers oder (ii) als in eine Trägermatrix eingebettete Pulver, in Form einer Beschichtung oder als Füllmaterial für eine Kavität im Implantat, vor. Variante (ii) kann herstellungstechnisch besonders einfach umgesetzt werden: Es wird ein gieß- oder sprühfähiges Gemisch aus der radioopaken Markerkomponente und dem als Trägermatrix agierenden Material, gegebenenfalls unter Zusatz eines Lösungsmittels, verarbeitet.

Die aus dem Stand der Technik bekannten biokorrodierbaren Metalllegierungen für medizinische Implantate besitzen im medizintechnischen Energiebereich von 60 - 100 keV nur eine geringe Röntgensichtbarkeit. Zur postoperativen Überwachung des Heilungsfortschrittes oder zur Kontrolle minimal-invasiver Eingriffe ist aber gerade die Röntgendiagnostik ein wichtiges Instrumentarium. So werden z. B. seit einigen Jahren Stents in den Koronararterien bei der akuten Myokardinfarkttherapie platziert. Der Stent wird im Bereich der Läsion der koronaren Gefäßwand positioniert und soll eine Obstruktion der Gefäßwand nach deren Aufweitung verhindern. Der Vorgang der Positionierung und Expansion der Stents muss während der Prozedur durch den Kardiologen laufend überwacht werden.

Bei Implantaten aus biokorrodierbaren metallischen Werkstoffen auf der Basis von Magnesium, Eisen oder Wolfram bestehen erhöhte Anforderungen an das Markermaterial:
- der Marker sollte durch die korrosiven Prozesse nicht frühzeitig vom Grundkörper des Implantates getrennt werden, um einer Fragmentbildung und damit der Gefahr der Embolisation vorzubeugen;
- der Marker sollte schon bei geringen Materialdicken eine hinreichende Röntgendichte besitzen und
- das Markermaterial sollte möglichst keinen oder allenfalls einen geringen Einfluss auf die Degradation des Grundkörpers haben.

Bei Einsatz von Markern aus metallischen Werkstoffen auf biokorrodierbaren metallischen Grundkörpern besteht jedoch das besondere Problem, dass sich aufgrund von elektrochemischen Wechselwirkungen zwischen den beiden metallischen Materialien die Degradation des Grundkörpers in einem Kontaktbereich zwischen Marker und Grundkörper ändert, d. h. in der Regel beschleunigt ist. DE 10 2008 043 642 A1 beschreibt eine Endoprothese mit einem voluminösen Marker, der mit einer Barriereschicht versehen ist, mittels der das radioopake Material des Markers gegenüber dem Grundkörper elektrisch isoliert ist. Der Grundkörper kann aus einer biokorrodierbaren Magnesiumlegierung bestehen.

### Zusammenfassung der Erfindung

Der vorliegenden Erfindung liegt die Aufgabe zugrunde, ein oder mehrere der zuvor angesprochenen Probleme des Standes der Technik zu beheben oder zumindest zu mindern. Die Erfindung betrifft ein Markerkomposit für medizinische Implantate aus einem biokorrodierbaren metallischen Werkstoff. Das Markerkomposit enthält eine Vielzahl von Partikeln aus einem radioopaken Metall, die in ein elektrisch nicht leitfähiges Polymer eingebettet sind. Die Partikel weisen eine zusätzliche elektrisch nicht leitfähige Beschichtung auf.

Der Erfindung liegt die Erkenntnis zugrunde, dass die Aufbringung einer passivierenden Schutzschicht auf den Metallpartikeln des Markers die Gefahr der Kontaktkorrosion weiter mindert bzw. völlig ausschließt.

Als radioopakes Metall für den Marker können alle herkömmlichen, im Zusammenhang mit Implantaten genannten radioopaken Metalle Einsatz finden. Der Markerkomponente liegt in der Trägermatrix in suspendierter Form vor. Das Metall ist vorzugsweise ein Element ausgewählt aus der Gruppe Gold, Iridium, Platin und Tantal. Insbesondere bestehen die Partikel aus Tantal.

Vorzugsweise haben die Partikel eine mittlere Teilchengröße von 0.2 µm bis 10 µm. Der Einsatz feinpulvriger Metallpartikel vereinfacht die Verarbeitung und Auftragung des Markerkomposits auf das Implantat beziehungsweise Einbringung des Markerkomposits in eine Kavität des Implantats. Die Trägermatrix verringert eine Kontaktfläche zwischen den metallischen Markerkomponenten und dem Grundkörper des Implantats, so dass unerwünschte Wechselwirkungen in Bezug auf das Korrosionsverhalten vermieden oder zumindest gemindert sind. Vorzugsweise wird der Bereich des Implantats, der den Marker tragen soll, mit einer kleinen Menge der Trägermatrix (oder mit einem anderen Polymer) vor Aufbringung des Markers beschichtet. Nach der Einbringung des Markerkomposits, kann die Kavität für eine zusätzliche Isolation mit einem Polymer noch mal beschichtet werden.

Vorzugsweise besteht die Beschichtung oder der Überzug der Partikel aus einem Carbid oder Oxid des radioopaken Metalls, z. B. Tantalcarbid oder Tantaloxid. Die Erzeugung von Oxidschichten bzw. Carbidschichten auf der Oberfläche der Metallpartikel ist hinlänglich bekannt und wird daher an dieser Stelle nicht näher ausgeführt. Das Oxidieren von Partikeln aus Ta kann durch eine ca. 1 bis 3-stündige Temperung zwischen 500 und 600°C an Luft realisiert werden. Durch die Reaktion mit Luftsauerstoff wird metallisches Tantal zu Tantal(V)-oxid oxidiert. Die niederen Oxide entstehen nur bei der Oxidation von Tantal oder Tantalverbindungen, und zwar bildet sich TaO*ₓ* (Ta₆O) bei etwa 300°C, TaO*_{y}* (Ta₄O) unterhalb von 500°C, TaO und Ta0₂ bilden sich bei der Oxidation des Tantals zu Ta₂0₅ als Zwischenschichten zwischen dem Metall und dem Ta₂O₅. Auf Grund der hohen Bildungsenthalpie von Tantaloxid ist die Wechselwirkung mit der umgebenden Körperflüssigkeit klein.

Diese kleine Wechselwirkung wird von den chemischen Reaktionen zwischen dem Mg-Implantat und der Körperflüssigkeit wesentlich überlagert und ist deshalb für die erfindungsgemäße Lösung bedeutungslos.

Alternativ kann die Beschichtung vorzugsweise aus Parylen bestehen.

Das Polymer der Trägermatrix ist vorzugsweise ausgewählt aus der Gruppe umfassend Polyurethane, Silikone, Polybutylmethacrylate, Cyanoacrylate und Epoxidharze.

Ein weiterer Aspekt der Erfindung liegt in der Bereitstellung eines medizinischen Implantats aus einem biokorrodierbaren metallischen Werkstoff, das einen Röntgenmarker aufweist, der aus einem Markerkomposit der vorgenannten Zusammensetzung besteht oder aus diesem Markerkomposit hergestellt ist. In letzterem Falle wird beispielsweise das Markerkomposit nach der Einbringung in eine Kavität oder Aufbringung als Beschichtung ausgehärtet.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defillibratoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird.

Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Biokorrodierbarer Legierungen umfassen biokorrodierbare Basislegierungen der Elemente Magnesium, Eisen, Zink, Molybdän und Wolfram. Bevorzugt ist Magnesium. Unter Basislegierung wird vorliegend ein metallisches Gefüge verstanden, dessen Hauptkomponente eines der Elemente Magnesium, Eisen, Zink, Molybdän und Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/1, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/1). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

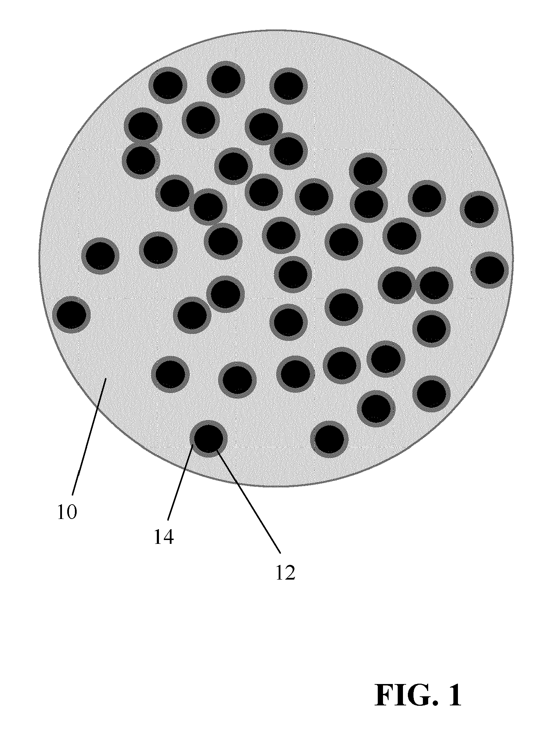

### Kurzbeschreibung der Figur

Die einzige Figur zeigt eine vergrößerte, schematische Schnittansicht eines Markers aus dem erfindungsgemäßen Markerkomposit.

### Detaillierte Beschreibung der Erfindung

Die Erfindung wird nachfolgend anhand eines Ausführungsbeispiels näher erläutert.

### Ausführungsbeispiel 1

In der einzigen Figur ist ein Schnitt von ca. 300 - 400 µm Durchmesser durch einen scheibenförmigen Marker dargestellt, der aus dem erfindungsgemäßen Markerkomposit besteht. Die Matrix 10 besteht aus einem Epoxidharz, in welches Tantalpartikel 12 von ca. 10µm Durchmesser eingelagert sind. Die Partikel 12 haben einen Gewichtsanteil von mehr als 80% am Marker. Die Partikel 12 weisen eine Tantal-Oxidschicht 14 an ihrer Oberfläche auf.

## Patentansprüche

1. Markerkomposit für medizinische Implantate aus einem biokorrodierbaren metallischen Werkstoff, wobei das Markerkomposit eine Vielzahl von Partikeln aus einem radioopaken Metall enthält, die in ein elektrisch nicht leitfähiges Polymer eingebettet sind, und wobei die Partikel eine zusätzliche elektrisch nicht leitfähige Beschichtung aufweisen.

2. Markerkomposit nach Anspruch 1, bei dem das radioopake Metall ein Element ausgewählt aus der Gruppe Gold, Iridium, Platin und Tantal ist.

3. Markerkomposit nach Anspruch 2, bei dem die Partikel aus Tantal bestehen.

4. Markerkomposit nach einem der vorhergehenden Ansprüche, bei dem die Beschichtung der Partikel aus einem Carbid oder Oxid des radioopaken Metalls besteht.

5. Markerkomposit nach einem der Ansprüche 1 bis 3, bei dem die Beschichtung der Partikel aus Parylen besteht.

6. Markerkomposit nach einem der vorhergehenden Ansprüche, bei dem das Polymer ausgewählt ist aus der Gruppe umfassend Polyurethane, Silikone, Polybutylmethacrylate, Cyanoacrylate und Epoxidharze.

7. Medizinisches Implantat aus einem biokorrodierbaren metallischen Werkstoff und mit einem Röntgenmarker, der aus einem Markerkomposit nach einem der Ansprüche 1 bis 6 besteht oder aus diesem hergestellt ist.

8. Medizinisches Implantat nach Anspruch 7, bei dem das Implantat ein Stent ist.

9. Medizinisches Implantat nach Anspruch 7 oder 8, bei dem der biokorrodierbare metallische Werkstoff eine Magnesiumlegierung ist.

10. Medizinisches Implantat nach einem der Ansprüche 7 bis 9, bei dem der Röntgenmarker in Form einer Beschichtung oder Kavitätenfüllung vorliegt.

## Claims

1. A marker composite for medical implants composed of a biocorrodible metal material, wherein the marker composite contains a plurality of particles formed from a radiopaque metal which are embedded in an electrically non-conductive polymer, and wherein the particles comprise an additional electrically non-conductive coating.

2. The marker composite according to Claim 1, in which the radiopaque metal is an element selected from the group consisting of gold, iridium, platinum, and tantalum.

3. The marker composite according to Claim 2, in which the particles consist of tantalum.

4. The marker composite according to one of the preceding claims, in which the coating of the particles consists of a carbide or oxide of the radiopaque metal.

5. The marker composite according to one of Claims 1 to 3, in which the coating of the particles consists of Parylene.

6. The marker composite according to one of the preceding claims, in which the polymer is selected from the group comprising polyurethanes, silicones, polybutyl methacrylates, cyanoacrylates, and epoxy resins.

7. A medical implant composed of a biocorrodible metal material and comprising an X-ray marker which consists of or is produced from a marker composite according to one of Claims 1 to 6.

8. The medical implant according to Claim 7, in which the implant is a stent.

9. The medical implant according to Claim 7 or 8, in which the biocorrodible metal material is a magnesium alloy.

10. The medical implant according to one of Claims 7 to 9, in which the X-ray marker is present in the form of a coating or cavity filling.

## Revendications

1. Marqueur composite pour implants médicaux constitué d'un matériau métallique bio-corrodable, où le marqueur composite contient une multiplicité de particules d'un métal radio-opaque qui sont incorporées dans un polymère électriquement non conducteur, et où les particules présentent un revêtement supplémentaire électriquement non conducteur.

2. Marqueur composite selon la revendication 1, chez lequel le métal radio-opaque est un élément choisi dans le groupe constitué par l'or, l'iridium, le platine et le tantale.

3. Marqueur composite selon la revendication 2, chez lequel les particules sont constituées de tantale.

4. Marqueur composite selon l'une des revendications précédentes, chez lequel le revêtement des particules est constitué par un carbure ou un oxyde du métal radio-opaque.

5. Marqueur composite selon l'une des revendications 1 à 3, chez lequel le revêtement des particules est constitué de parylène.

6. Marqueur composite selon l'une des revendications précédentes, chez lequel le polymère est choisi dans le groupe comprenant les polyuréthanes, les silicones, le polybutylméthacrylates, les cyanoacrylates et les résines époxydes.

7. Implant médical à base d'un matériau métallique bio-corrodable et avec un marqueur pour rayons X qui est constitué d'un marqueur composite selon l'une des revendications 1 à 6 ou est fabriqué à partir de celui-ci.

8. Implant médical selon la revendication 7, chez lequel l'implant est un stent.

9. Implant médical selon les revendications 7 ou 8, chez lequel le matériau métallique bio-corrodable est un alliage de magnésium.

10. Implant médical selon l'une des revendications 7 à 9, chez lequel le marqueur des rayons X se présente sous la forme d'un revêtement ou d'un remplissage de cavités.
